# EUROPÄISCHE PATENTSCHRIFT

(11) **EP 2 129 434 B1**
(45) Veröffentlichungstag und Bekanntmachung des Hinweises auf die Patenterteilung: **27.10.2010**
(21) Anmeldenummer: 08734903.1
(22) Anmeldetag: 31.03.2008
(51) Int. Cl.: A61N 5/06, H01J 61/16

(54) **BRÄUNUNGSVORRICHTUNG ZUR BRÄUNUNG DER MENSCHLICHEN HAUT**
TANNING DEVICE FOR TANNING HUMAN SKIN
DISPOSITIF DE BRONZAGE POUR LE BRONZAGE DE LA PEAU HUMAINE

(30) Priorität: 30.03.2007 DE 102007015740
(43) Veröffentlichungstag der Anmeldung: 09.12.2009
(73) Patentinhaber: Schwellenbach, Christoph, 53721 Siegburg (DE)
(72) Erfinder: Schwellenbach, Christoph, 53721 Siegburg (DE)
(74) Vertreter: Gesthuysen, von Rohr & Eggert
(86) Internationale Anmeldenummer: PCT/EP2008/002545
(87) Internationale Veröffentlichungsnummer: WO 2008/119536

(56) Entgegenhaltungen:
- EP-A- 0 581 354
- EP-A- 1 733 691
- WO-A-03/105185
- DE-A1- 10 240 922
- DE-A1-102005 003 790
- DE-U1- 29 514 036
- US-A- 2 797 363

## Beschreibung

Die Erfindung betrifft eine Bräunungsvorrichtung zur Bräunung der menschlichen Haut nach dem Oberbegriff des Anspruchs 1.

Moderne Bräunungsvorrichtungen zur Bräunung der menschlichen Haut enthalten in der Regel zwei verschiedene Strahlersysteme, nämlich einerseits Bräunungsröhren, die auch Niederdrucklampen genannt werden, und spezielle Brenner, vor allem im Gesichtsbereich, die als Hochdruckbrenner bezeichnet werden. Sowohl bei den Niederdrucklampen als auch bei den Hochdrucklampen handelt es sich um Lichtquellen, welche zur Lichterzeugung entweder die spontane Emission durch atomare oder molekulare elektronische Übergänge oder aber die Rekombinationsstrahlung eines durch elektrische Entladung erzeugten Plasmas ausnutzen.

Aus der DE-U-295 14 036 geht beispielsweise ein Bestrahlungsapparat hervor, bei dem als Strahlungsquelle eine Hochdruckmetallhalogenidlampe vorgesehen ist. Die Metallhalogenidlampe weist ein zylindrisches Brennerrohr mit zwei gegenüberliegenden Elektroden auf. Im Brennerrohr befindet sich eine Füllung, die Quecksilber, Halogene und weitere Materialien enthält. Bestrahlungsgeräte der vorgenannten Art werden üblicherweise mit einem konventionellen Vorschaltgerät (KVG) betrieben, wobei die Lampenspannung üblicherweise zwischen 100 und 150 Volt beträgt. Im Strompfad liegt das Zündgerät, welches so lange Zündimpulse liefert, bis die Lampe zündet. Nach dem Zündvorgang der Lampe muss sich die Füllung im Brennerrohr erhitzen, bis sie verdampft. Die Füllung ist beim Startvorgang nicht ionisiert und hat einen sehr hohen Widerstand, der eine hohe Zündspannung benötigt, damit ein Lichtbogen zwischen den beiden Elektroden entsteht. Im Lichtbogen erreicht zunächst das Quecksilber bei ca. 356° C seinen Siedepunkt und fängt mit der Lichtemission an. Bei fortschreitender Erwärmung sieden zeitlich verzögert auch die anderen Inhaltsstoffe. Während dieses Vorgangs kommt die Lampe nur sehr langsam auf ihren optimalen Betriebszustand. Das Hochbrennen der Lampe bis zu ihrem optimalen Betriebszustand ist für den Nutzer deutlich erkennbar und wird als störend empfunden.

Wird die bekannte Lampe ausgeschaltet, so muss sie erst wieder abkühlen, um einen Neustart zu ermöglichen. Werden Lampen der vorgenannten Art bei Sonnenbänken eingesetzt, übernehmen in der Regel starke Lüfter diese Aufgabe. Trotz der Kühlung über die Lüfter ist es häufig nicht möglich, die Lampe direkt nach dem Ausschalten wieder einzuschalten. In der Regel ist eine Kühlphase von mehr als einer Minute erforderlich, was insbesondere im gewerblichen Betrieb nachteilig ist.

Lampen der vorgenannten Art haben üblicherweise eine Lebensdauer zwischen 500 bis 1000 Stunden. Dies liegt u.a. daran, dass die Lampen aufgrund der relativ hohen Wärmeabgabe starken Beanspruchungen unterliegen. Die hohe Wärmeabgabe wirkt sich jedoch nicht nur nachteilig auf die maximale Betriebsdauer aus, sondern ist auch für den Nutzer unangenehm, da dies zu einem starken Schwitzen beim Bräunungsvorgang führt. Darüber hinaus sind derartige Lampen vergleichsweise groß, so dass entsprechend große Reflektoren erforderlich sind. Die großen Reflektoren führen zu großen Brennweiten, einer größeren Streustrahlung und damit einer relativ geringen Effizienz im UV-Strahlungsbereich.

Aus der US 2,797,363 geht eine elektrische Entladungslampe zur Bräunung der Haut, die das Sonnenlicht nachbilden soll hervor. Die bekannte Lampe ist eine Gasentladungslampe, bei der die Elektroden durch einen Wendel überbrückt werden, wobei das Gas Xenon sein kann. Ein weiterer Glühwendel ist mit den Elektroden in Serie geschaltet. Die Lampe sendet Strahlung im Spektralbereich zwischen 298 und 440 nm aus, wobei ein kleiner Anteil der gesamten Strahlung, der zur Rötung und Pigmentation der Haut führt, zwischen 298 und 320 nm liegt und wobei die Strahlung zwischen 320 und 440 nm die Bräunung der Haut beeinflusst.

Aus der EP 0 581 354 A1 geht eine Hochdruckgasentladungslampe hervor, deren Brennkammer mit Quecksilber, Natriumjodid, Scandiumjodid und Xenon gefüllt ist. Der Xenondruck beträgt 7 bar bei Raumtemperatur. Die Lampe weist weiterhin einen die Brennkammer umhüllenden Hüllkolben auf.

In der EP 1 733 691 A1 wird ein Gerät zur kosmetischen Hauterneuerungsbehandlung (Phototherapie), die eine Hochintensitäts-Xe-Entladungslampe mit mindestens einem dominanten Peak zwischen 550 und 700 nm aufweist, offenbart. Darüber hinaus kann eine solche Lampe mit mindestens einem dominanten Peak zwischen 390 und 430 nm betrieben werden. Das Spektrum kann durch Zugabe von Halogensalzen eingestellt werden. Weiterhin können der Lampe Metallhalogenide zur Stabilisierung des Lichtbogens zugesetzt werden.

Die DE 102 40 922 A1 offenbart eine Bestrahlungsanordnung zur Behandlung von zellvermittelten Entzündungen der Haut. In einer Ausführungsform dient eine Xe-Blitzlampe als Strahlungsquelle, die im Bereich von 200 bis 2000 nm emittiert. Die nicht gewünschten Spektralbereiche werden herausgefiltert. Zur Erhöhung des gewünschten blauen Spektralbereiches werden Halogenide in die Xe-Blitzlampe gefüllt. Dotierung der Lampe mit Quecksilber, Quecksilberjodid oder Amalgan erhöht den Wirkungsgrad im blauen Bereich.

Die WO 03/105185 A betrifft eine Niederdruck-Quecksilberdampfentladungslampe, die Licht des elektromagnetischen Spektrums in wählbaren Wellenlängenbereichen emittiert. Die Farbtemperatur der Lampe ist dimmbar ausgeführt

Die DE 10 2005 003 790 A1 betrifft ein Bestrahlungsgerät zur Bräunung der Haut mit UV-Niederdrucklampen, wobei eine Filterscheibe eine Strahlungsdurchgangsöffnung abdeckt. Die Filterscheibe kann eine Beschichtung aufweisen, die als Spiegel wirkt. Das sichtbare Licht von 410 bis 700 nm wird nicht durchgelassen, sondern reflektiert. UV-Strahlung bis 410 nm passiert die Filterscheibe.

Aufgabe der vorliegenden Erfindung ist es, eine Bräunungsvorrichtung zur Bräunung der menschlichen Haut zur Verfügung zu stellen, bei der die Effizienz und damit die Lichtausbeute erhöht ist.

Zur Lösung der vorgenannten Aufgabe sind nun die im Anspruch 1 angegebenen Merkmale vorgesehen. Wenngleich Xenon-Gasentladungslampen als solche grundsätzlich bereits bekannt sind, ist überraschenderweise festgestellt worden, dass sich derartige Lampen mit einer Quecksilber und Metallsalze enthaltenden Füllung ausgezeichnet als Bräunungslampen zur Bräunung der menschlichen Haut im UV/A- und UVB-Bereich eignen. Bei den erfindungsgemäßen Bräunungsgeräten handelt es sich dabei insbesondere um solche, die zu kosmetischen Zwecken eingesetzt werden, beispielsweise in Sonnenstudios oder in privaten Haushalten. Eine Xenon-Gasentladungslampe als Bräunungslampe bietet eine Vielzahl von Vorteilen. Xenon dient bei der erfindungsgemäßen Gasentladungslampe als Startgas, um direkt nach dem Einschalten ausreichend Licht zu liefern. Die Xenonentladung verdampft dann das in der Füllung enthaltene flüssige Quecksilber, welches bei Betriebstemperatur zumindest den wesentlichen Teil des Entladungsgases bildet. Aufgrund dieses Umstandes ist, anders als bei den bekannten Hochdruckmetallhalogenidlampen, ein direkter Warmstart möglich. Darüber hinaus zeichnet sich die Xenon-Gasentladungslampe durch einen niedrigen Energieverbrauch aus, was gerade bei Bräunungsgeräten, die dauerhaft mit einer hohen elektrischen Leistung betrieben werden, von erheblicher Bedeutung ist. Da Xenon-Gasentladungslampen im Übrigen sehr kleinbauend sind und ein eher punktförmiges Licht erzeugen, ergeben sich gerade bei Hautbräunungsvorrichtungen weitere wesentliche Vorteile. Die Xenon-Gasentladungslampe hat eine sehr kleine Brennweite. Bedingt durch die kleine Brennweite der Lampe kann der Reflektor der Hautbräunungsvorrichtung in seiner Geometrie höchst effizient ausgelegt werden. Die Streustrahlung im Reflektor wird minimiert und der Strahlungsweg gegenüber bekannten Systemen verringert, so dass sich eine hohe Effizienz im UV-Strahlungsbereich ergibt.

Ein weiterer Vorteil der Xenon-Gasentladungslampe besteht darin, dass im Verhältnis zu Hochdruckmetallhalogenidlampen eine erheblich verringerte Wärmeabgabe auftritt, woraus sich letztlich auch die deutlich höhere Betriebsdauer der erfindungsgemäßen Lampe gegenüber den im Stand der Technik verwendeten Lampen ergibt. Die mittlere Lebensdauer liegt bei ca. 2000 Stunden. Da die erfindungsgemäße Lampe eine deutlich höhere Lichtausbeute als die üblichen Halogenlampen hat, lässt sich ein geringerer Energieverbrauch bei gleicher Leistung wie übliche Lampen erreichen.

Die erfindungsgemäße Lampe weist bevorzugt ein Strahlungsspektrum auf, bei dem dominante Spektrallinien im UV/A- und im UVB-Bereich und bevorzugt auch im Blaulichtbereich vorgesehen sind. Dabei bezeichnen dominante Spektrallinien solche Frequenzbereiche, in denen erheblich mehr Energie abgegeben wird, als in anderen Bereichen. Bei der erfindungsgemäßen Lampe ist es bevorzugt so, dass mit Ausnahme des Wellenlängenbereichs zwischen 520 nm bis 550 nm die höchsten Spektrallinien bzw. die Spektralspitzen ausschließlich im UV/A- und insbesondere im WB-Bereich und bevorzugt auch im Blaulichtbereich insbesondere bis ca. 450 nm liegen. Dabei werden wenigstens mehr als 30 %, vorzugsweise mehr als 40 % und insbesondere mehr als 50 % der Strahlung in diesen Bereich zur Bräunung emittiert. Letztlich ist es so, dass zwischen 60 % und 90 % der abgegebenen Strahlungsenergie bzw. -intensität im Wellenlängenbereich zwischen 280 nm und 450 nm liegen. Mit dieser Strahlungsmenge ist der Strahlungsanteil bzw. die Energie gemeint, der letztlich auf den Benutzer während der Bräunung wirkt.

Im übrigen ist im Zusammenhang mit der vorliegenden Erfindung festgestellt worden, dass sich nicht nur die UV/B-Strahlung im Wellenlängenbereich zwischen 280 bis 315 nm und die UV/A-Strahlung im Wellenlängenbereich zwischen 315 und 380 nm zur Bräunung der menschlichen Haut eignet, sondern auch die Blaulichtstrahlung im Wellenlängenbereich zwischen 380 und 500 nm und insbesondere im Bereich bis ca. 450 nm. Bisher war man der Auffassung, dass sich die Blaulichtstrahlung an sich nur zu medizinischen Behandlungszwecken, wie beispielsweise bei Akne, eignet. Damit hat die bei der erfindungsgemäßen Lampe emittierte Strahlung nicht nur einen Bräunungs-, sondern durch den Blaulichtstrahlungsanteil sowohl einen ergänzenden Bräunungs- und auch einen medizinischen Effekt.

Zum optimalen Betrieb der erfindungsgemäßen Bräunungslampe ist diese derart ausgelegt, dass der Betriebsdruck zwischen 25 und 100 bar, insbesondere zwischen 35 und 50 bar liegt. Dabei ist der Gasfülldruck der Bräunungslampe im ausgeschalteten Zustand größer als 2 bar und liegt vorzugsweise zwischen 5 und 15 bar und insbesondere zwischen 6 und 10 bar.

Im Übrigen ist die erfindungsgemäße Lampe bevorzugt derart ausgelegt, dass zur Zündung des Lichtbogens der Bräunungslampe ein Hochspannungsimpuls von mehr als 15 kV, vorzugsweise zwischen 20 und 40 kV erforderlich ist. Die Betriebsspannung sinkt anschließend erheblich ab und liegt in der Regel zwischen 10 und 130 Volt. Hierbei handelt es sich dann in der Regel um eine Rechteckspannung, die üblicherweise eine Frequenz von mehr als 50 Hz, insbesondere zwischen 200 und 600 Hz aufweist.

Zur Steuerung des Zündens und des Betriebes der erfindungsgemäßen Lampe dient ein regelbares oder nicht regelbares elektronisches Vorschaltgerät (EVG). Das elektronische Vorschaltgerät dient im Wesentlichen zur Strombegrenzung, da ansonsten der Entladungsstrom immer weiter ansteigen würde, bis die Lampe zerstört wird oder die Sicherung anspricht.

Von besonderem Vorteil in Verbindung mit dem elektronischen Vorschaltgerät ist, dass diese eine Dimmungseinrichtung aufweist, so dass es möglich ist, die Bräunungslampe mittels des elektronischen Vorschaltgeräts zu dimmen.

Zum Schutz der Brennkammer gegen äußere Einflüsse und im übrigen zur Wärmeisolation kann die Brennkammer in einem äußeren, vorzugsweise evakuierten Hüllkolben vorgesehen bzw. angeordnet sein. Der Hüllkolben verhindert im Übrigen bei einem eventuellen Bersten der Brennkammer ein ungewolltes Austreten des Quecksilbers. Damit eine UV-Bestrahlung des Nutzers möglich ist, versteht es sich, dass der Hüllkolben, wie die Brennkammer auch, eine mehr als 20%ige, vorzugsweise mehr als 40%ige und insbesondere mehr als 60%ige Durchlässigkeit für UV-Strahlung aufweist. Letztlich sollte der Hüllkolben die gleiche hohe Durchlässigkeit für UV-Strahlung haben wie die Brennkammer, wobei die Durchlässigkeit vorzugsweise zwischen 50% und 100% liegen sollte. Dabei ist jeder Einzelwert in dem vorgenannten Intervall möglich, ohne dass es einer ausdrücklichen Erwähnung bedarf.

Um eine hohe Lichtausbeute im geforderten UV-Bereich bereitzustellen, besteht das Material der Brennkammer und/oder des Hüllkolbens aus UVdurchlässigem Quarzglas. Das Quarzglas wird auch aufgrund seiner mechanischen und thermischen Stabilität bevorzugt eingesetzt. Der Quarztyp richtet sich dabei nach der für den erfindungsgemäßen Anwendungszweck notwendigen hohen UV-Intensität. Um einen hohen UV-A- und einen bestimmten UV-B-Anteil bei gleichzeitiger Vermeidung bzw. Verringerung des UV-C-Anteils zu erzielen, kann die Brennkammer und/oder der Hüllkolben eine entsprechende, gegebenenfalls teilweise Beschichtung und/oder Oberflächenbearbeitung zur entsprechenden Filterung aufweisen. Die Brennkammer und/oder Hüllkolben können auch teilweise (insbesondere halbseitig) beschichtet und so als Reflektor ausgeführt sein.

Die erfindungsgemäße Bräunungslampe zeichnet sich durch einen im Vergleich zur Hochdruckmetallhalogenidlampe extrem kleinen Brennraum in der Brennkammer aus. Dabei ist die Brennkammer bevorzugt ellipsoidförmig ausgebildet und insbesondere rotationssymmetrisch. Im Hinblick auf den sehr hohen Betriebsdruck bietet sich insbesondere eine kugelartige Form der Brennkammer und/oder des darin befindlichen Brennraums aus. Allerdings versteht es sich, dass auch andere, insbesondere rotationssymmetrische Ellipsoidformen möglich sind. Bei einer kugelförmigen Brennkammer soll der Innendurchmesser zwischen 2 bis 10 mm und vorzugsweise im Bereich zwischen 5 und 6 mm liegen. Die Verwendung einer derart kleinen Brennkammer hat den Vorteil, dass sich quasi eine punktförmige Lichtquelle ergibt. Eine derartige punktförmige Lichtquelle ist, wenn sie in den Reflektor einer Bräunungsvorrichtung eingesetzt ist, ausgesprochen effektiv, da bei entsprechender Reflektorauslegung die Streustrahlung minimiert und die dem Nutzer bei der Bräunung zugeführte Strahlungsmenge optimiert wird. Letztlich ergeben sich kaum Strahlungsverluste durch Streustrahlung.

Die Anode und die Kathode der erfindungsgemäßen Lampe bestehen vorzugsweise aus Wolfram und sind insbesondere mit Thorium dotiert, um die Elektronenemissionen zu verstärken. Dabei kann die Kathode klein und spitz sein, damit die Spitze die für eine effiziente Elektronenemission erforderliche hohe Temperatur erreicht. Die Anode kann massiver sein, damit sie den Elektronenbeschuss aushalten und die entstehende Wärme wirksam ableiten kann. Grundsätzlich können an einer oder beiden Elektroden Wicklungen vorgesehen sein, um die Ausbildung des Lichtbogens zu unterstützen.

Im Zusammenhang mit der vorliegenden Erfindung ist festgestellt worden, dass der lichte Abstand der Elektroden mehr als 1 mm betragen sollte. Bevorzugt liegt der Elektrodenabstand zwischen 2 und 15 mm und insbesondere zwischen 3 und 10 mm.

Im Zusammenhang mit der vorliegenden Erfindung ist festgestellt worden, dass die Füllung der Brennkammer neben dem Startergas Xenon und dem eigentlichen Entladungsgas Quecksilber Natriumjodid und/oder Scandiumjodid als Metallsalze aufweisen kann.

Um die Lebensdauer der erfindungsgemäßen Bräunungslampe zu erhöhen, ist vorzugsweise eine Kühlung mittels einer entsprechenden Kühleinrichtung an der Bräunungsvorrichtung vorgesehen. Die Kühlung kann beispielsweise über einen Lüfter erfolgen, der der Bräunungslampe permanent Umgebungsluft zuführt.

Zur Erzielung homogener Strahlungsverhältnisse auf der Haut des zu Bräunenden ist eine entsprechende Fokussierung der Strahlung erforderlich. Dies kann durch entsprechend bearbeitete Oberflächen einer vor den Reflektor gesetzten Schutzscheibe genauso erfolgen, wie durch entsprechend vorgesetzte Optiken. Die Oberfläche kann beispielsweise durch eine entsprechende Aufrauhung oder sonstige Bearbeitung satiniert sein. Die fokussierte Strahlung kann aber auch auf Reflektoren gebracht werden, welche durch die Art der Geometrie, der Oberflächenbeschaffenheit und/oder der Beschichtung das gewünschte Bestrahlungsfeld erzeugt.

Die erfindungsgemäße Bräunungslampe kann im Übrigen entweder an einem Ende oder an beiden Enden ein Anschlußsockel aufweisen, der bzw. die in entsprechende Halterungen an der Bräunungsvorrichtung einsetzbar sind. Der oder die Metallsockel sorgen letztlich also für den äußeren elektrischen Anschluss und die mechanische Halterung.

Im Übrigen kann die erfindungsgemäße Bräunungsvorrichtung sowohl als Sonnenbank beispielsweise in Form eines Tunnelgeräts oder aber als Stand- bzw. Tischgerät ausgebildet sein.

Hinzuweisen ist darauf, dass sämtliche der vorgenannten und auch nachfolgenden Bereichsangaben und Intervalle alle Zwischenbereichsangaben und Zwischenintervalle sowie alle Einzelwerte enthalten, die innerhalb der jeweiligen Intervallgrenzen angegeben sind. Alle Zwischenwerte und Zwischenintervalle gelten als erfindungswesentlich, auch wenn diese im Einzelnen nicht angegeben sind. Dies gilt insbesondere für die im Wellenlängenbereich zwischen 280 und 500 nm emittierte Strahlungsenergie, die zwischen 30 und 90 % der insgesamt von der erfindungsgemäßen Lampe emittierten Strahlung liegen kann. Zwischen den Bereichsgrenzen ist dabei jeder Einzelwert und auch jedes Zwischenintervall, auch im Dezimalbereich, möglich. Damit sind beispielsweise Werte wie 31 %, 32 % ... 88 %, 89 % möglich.

Nachfolgend werden Ausführungsbeispiele der Erfindung anhand der Zeichnung erläutert. Dabei bilden alle beschriebenen Merkmale für sich oder in beliebiger Kombination den Gegenstand der vorliegenden Erfindung, unabhängig von der nachfolgenden Beschreibung der Ausführungsbeispiele an sich.

Es zeigt
- Fig. 1: eine perspektivische Ansicht einer erfindungsgemäßen Bräu- nungsvorrichtung,
- Fig. 2: eine schematische Ansicht eines Teils der Bräunungsvorrichtung aus Fig. 1,
- Fig. 3: eine Ansicht einer anderen Ausführungsform einer erfindungs- gemäßen Bräunungsvorrichtung,
- Fig. 4: eine schematische Darstellung einer Bräunungslampe der erfin- dungsgemäßen Bräunungsvorrichtung,
- Fig. 5: eine andere Ausführungsform einer erfindungsgemäßen Bräu- nungslampe und
- Fig. 6: eine grafische Darstellung des Spektrums der Strahlung der Bräunungslampe der erfindungsgemäßen Bräunungsvorrichtung.

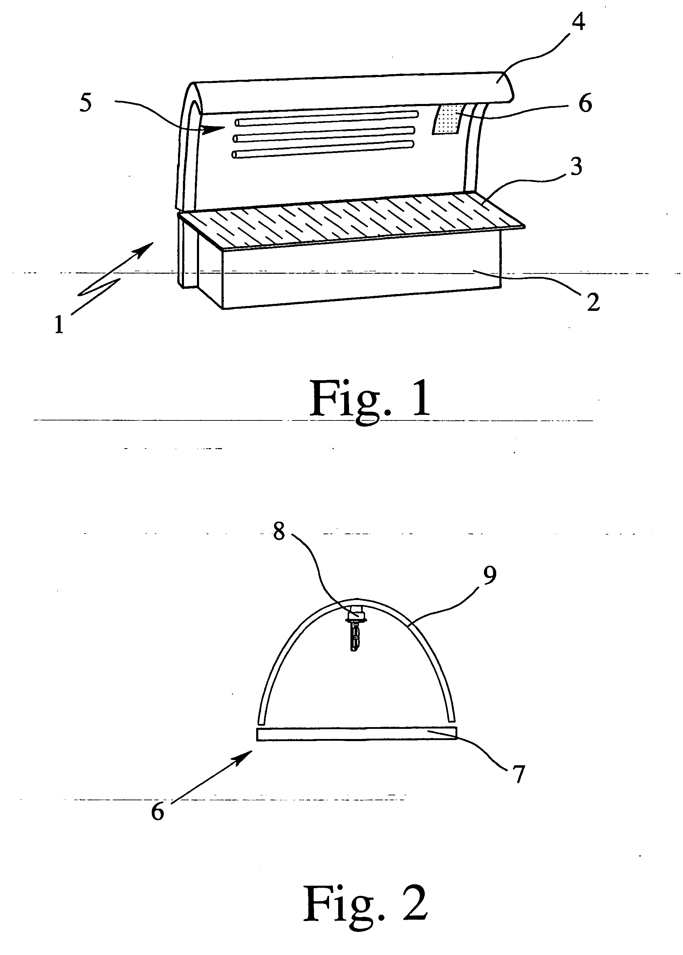

In Fig. 1 ist eine als Sonnenbank ausgebildete Bräunungsvorrichtung 1 zur UV-Bestrahlung der menschlichen Haut dargestellt. Bei der Bräunungsvorrichtung 1 handelt es sich um ein sogenanntes Tunnelgerät, das einen Unterbau 2 mit Liegefläche 3 und ein am Unterbau 2 angelenktes, verschwenkbares Oberteil 4 aufweist. Das Oberteil 4 ist auf den Unterbau 2 herabschwenkbar, so dass sich ein Tunnel ergibt, in dem sich während sich des Betriebes der Benutzer befindet. Unterhalb der Liegefläche und im Oberteil 4 befinden sich vorliegend langgestreckte Leuchtstoff-Bräunungsleuchten 5. Hinter den Bräunungsleuchten befinden sich jeweils Reflektoren, die im Einzelnen nicht dargestellt sind.

Im Gesichtsbereich des Oberteils 4 der Bräunungsvorrichtung 1 befindet sich ein Gesichtsbräuner 6. Der Gesichtsbräuner 6 weist eine äußere Schutzscheibe 7, wenigstens eine als Gasentladungslampe ausgebildete Bräunungslampe 8 und einen äußeren Reflektor 9 auf.

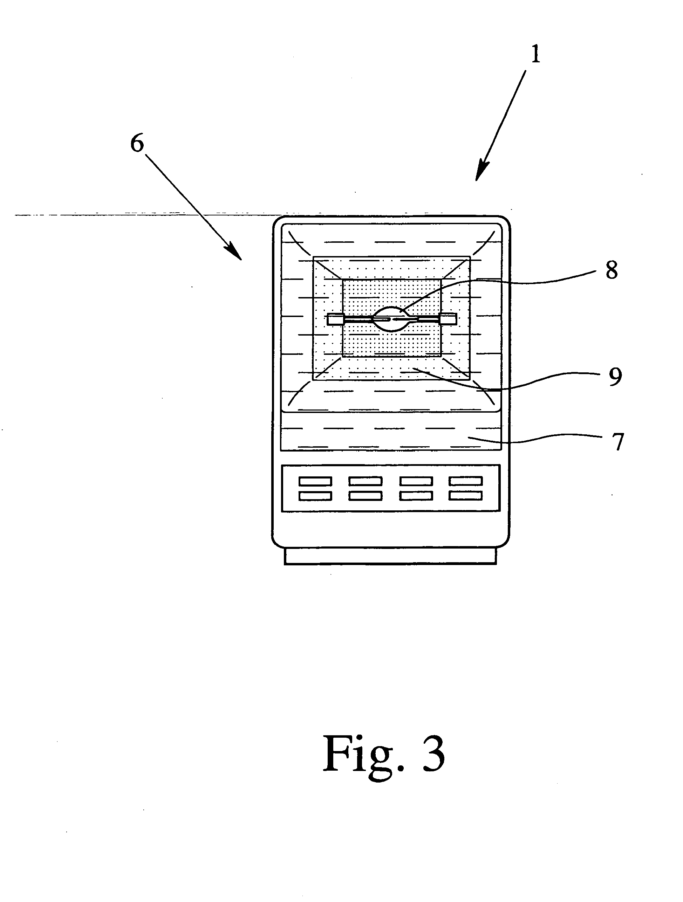

In Fig. 3 ist eine als Tischgerät ausgebildete Bräunungsvorrichtung 1 dargestellt. Dieses Gerät weist ebenfalls einen Gesichtsbräuner 6 mit einer äußeren Schutzscheibe 7, einer Bräunungslampe 8 und einem Reflektor 9 auf.

Im übrigen versteht es sich, dass vorliegend lediglich zwei Alternativen von Bräunungsvorrichtungen 1 dargestellt sind, es jedoch auch noch andere Ausfiihrungsformen von Bräunungsgeräten gibt, und die vorliegende Erfindung nicht auf die dargestellten Alternativen beschränkt ist.

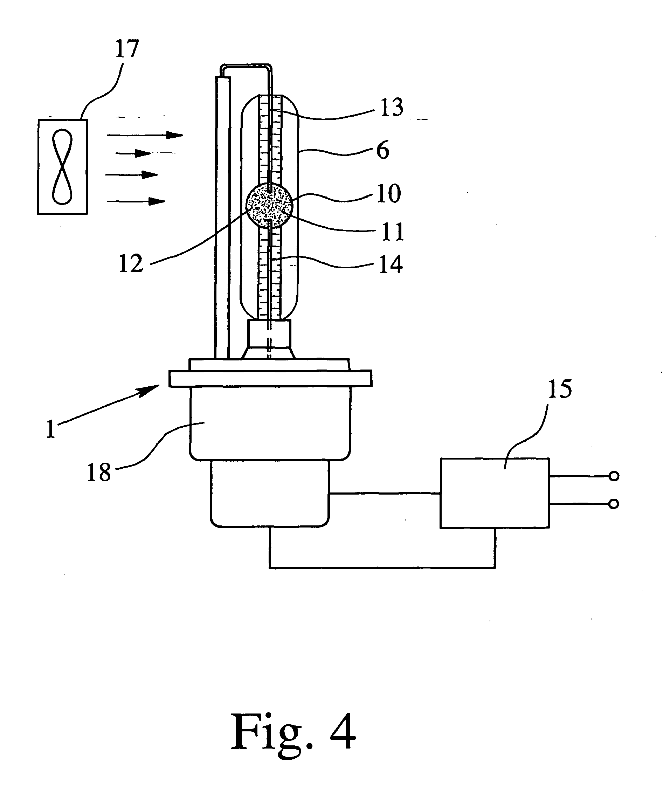

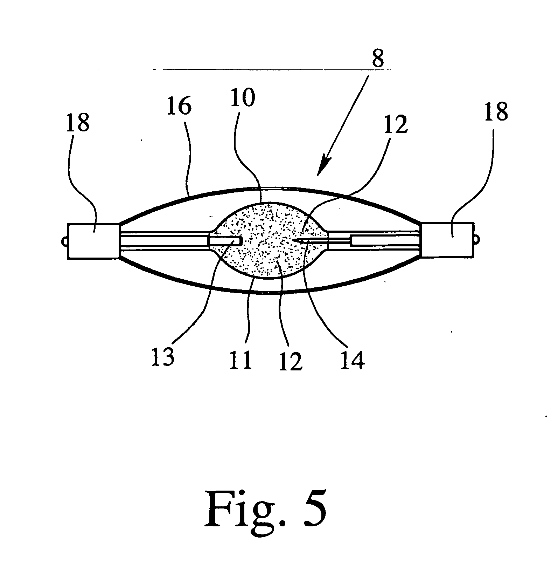

Wie sich insbesondere aus den Fig. 4 und 5 ergibt, weist die Bräunungslampe 8 eine Brennkammer 10 mit einem innerhalb der Brennkammer 10 liegenden Brennraum 11 auf. Im Brennraum 11 befindet sich ein Füllgas 12. Das Füllgas 12 dient zur Erzeugung eines Lichtbogens. In die Brennkammer 10 ragen zwei Elektroden 13, 14 hinein. Bei der Elektrode 13 handelt es sich vorliegend um die Anode, bei der Elektrode 14 um die Kathode. Im Übrigen besteht die Brennkammer 12 aus einem Material, das eine UV-Durchlässigkeit von mehr als 20 % aufweist. Dabei ist jeder Wert zwischen 20% und 100% möglich. Bevorzugt weist das Material der Brennkammer eine sehr hohe UV-Durchlässigkeit auf.

Wesentlich ist nun, dass die Bräunungslampe 8 als Xenon-Gasentladungslampe ausgebildet ist und im Betrieb einen Betriebsdruck von in jedem Falle mehr als 20 bar aufweist. Vorliegend beträgt der Betriebsdruck etwa bis zu 40 bar, wobei der Gasfülldruck im ausgeschalteten Zustand bei ca. 8 bar liegt. Die Zündung des Füllgases 6 zur Erzeugung des Lichtbogens erfolgt durch das Anlegen einer Zündspannung zwischen 20 und 40 kV. Die Steuerung des Zündvorgangs und des anschließenden Betriebes erfolgt über ein in Fig. 4 schematisch dargestelltes elektronisches Vorschaltgerät 15, das mit der Bräunungslampe 8 gekoppelt ist. Über das elektronische Vorschaltgerät 15 ist die Bräunungslampe 8 im Übrigen auch bedarfsweise zu dimmen. Hierzu sind eine entsprechende Ansteuerung und Regelung sowie wenigstens ein entsprechender Schalter vorgesehen.

Wie sich insbesondere aus den Fig. 4 und 5 ergibt, befindet sich die Brennkammer 10 in einem Hüllkolben 16. Der Hüllkolben 16 ist vorliegend evakuiert. Der Hüllkolben 16 weist wie auch die Brennkammer 10 eine hohe Durchlässigkeit für UV-Strahlung auf. Bei dem dargestellten Ausführungsbeispiel handelt es sich bei dem Material der Brennkammer 10 und des Hüllkolbens 16 um UV-durchlässiges Quarzglas. Durch entsprechende Materialwahl und/oder Beschichtung und/oder Oberflächenbearbeitung können dabei bestimmte gewünschte Spektren bzw. UV-A- und/oder UV-B-Anteile erzielt werden. Je nach Art der Oberflächenbearbeitung und/oder Beschichtung kann auch Einfluss auf sichtbare und Infrarotstrahlung genommen werden.

Bei dem in Fig. 4 dargestellten Ausführungsbeispiel ist die Brennkammer 10 etwa kugelförmig ausgebildet, während sie bei der in Fig. 5 dargestellten Ausführungsform in Form eines rotationssymmetrischen Ellipsoids ausgebildet ist. Der Abstand der Elektroden 13, 14 liegt in beiden Fällen zwischen 4 und 5 mm. Der Innendurchmesser der in Fig. 4 dargestellten kugelförmigen Brennkammer 10 liegt zwischen 5 und 6 mm. Die Elektroden 13, 14 selbst bestehen aus Wolfram, wobei bei der in Fig. 5 dargestellten Ausführungsform die Kathode an ihrem vorderen Ende angespitzt und eher dünner ist, während die Anode an ihrem vorderen Ende eher gerundet und im Übrigen dicker ist.

Die Füllung 12 selbst besteht vorliegend überwiegend aus Xenon und einem kleinen Anteil an Quecksilber. Des Weiteren sind kleine Anteile an Natriumjodid und Scandiumjodid vorgesehen.

Im Übrigen ist in Fig. 4 schematisch dargestellt, dass der Bräunungslampe 8 eine Kühleinrichtung 17 zugeordnet ist. Im vorliegenden Fall handelt es sich bei der Kühleinrichtung 17 um einen Axiallüfter, wobei es sich versteht, dass auch andere Kühler eingesetzt werden können.

Die in Fig. 4 dargestellte Bräunungslampe 8 weist nur an ihrem einen Ende einen Anschlußsockel 18 auf. Bei der in Fig. 5 dargestellten Bräunungslampe 8 ist an jedem Ende ein Anschlußsockel 18 vorgesehen. Dabei ist bei der in Fig. 5 dargestellten Ausführungsform der Hüllkolben 16 an den Anschlußsockeln 18 befestigt. Im Übrigen ist es bei der in Fig. 2 dargestellten Ausführungsform so, dass die Bräunungslampe 8 senkrecht angeordnet ist, während bei der in Fig. 3 dargestellten Ausführungsform eine horizontale Anordnung vorgesehen ist.

Die Funktionsweise der erfindungsgemäßen Bräunungslampe 8 sieht derart aus, dass mit einem vom elektronischen Vorschaltgerät 15 erzeugten Hochspannungsimpuls ein Funke erzeugt wird, der das in der Brennkammer 10 befindliche Füllgas 12, das an sich elektrisch nicht leitend ist, ionisiert und dadurch letztlich einen leitfähigen Tunnel zwischen den Elektroden 13, 14 schafft. Durch diesen Tunnel wird der elektrische Widerstand klein und es fließt ein Strom zwischen den Elektroden 13, 14. Der Strom regt das in der Füllung enthaltene Xenon zu Lichtemissionen an. Nach der Zündung kann die Bräunungslampe 8 mit Nennleistung oder höherer Leistung, insbesondere mit kontrollierter Überlast betrieben werden. So kann die Zeit bis zum Erreichen der optimalen Betriebslage (Betriebszustand) beeinflusst werden. Durch den mit höherer Leistung betriebenen Lichtbogen steigt die Temperatur im Kolben rasch an und das Quecksilber beginnt zu verdampfen. Dadurch ändert sich etwas die Lichtfarbe. Der Dampfdruck in der Lampe und die Lichtabgabe nimmt zu. Außerdem sinkt der Widerstand zwischen den Elektroden 13, 14, was von dem elektronischen Vorschaltgerät 15 erkannt und entsprechend geregelt wird. Schon in dieser Anlaufphase dominiert das Quecksilber-Spektrum der abgegebenen Strahlung. Wenn das Quecksilber und die etwaig vorhandenen Metallsalze sich in der Dampfphase befinden, hat der Lichtbogen seine endgültige Form erreicht und die Lichtausbeute ihren Sollwert. Das elektronische Vorschaltgerät 15 steuert die zugeführte elektrische Leistung und hält diese stabil, damit der Lichtbogen nicht flackert. Je nach Ansteuerung und Regelung des elektronischen Vorschaltgeräts 15 kann das Erreichen der vollen UV-Ausbeute in wenigen Sekunden erreicht werden.

Die Zündung der Lampe erfolgt bei einem Hochspannungsimpuls von bis zu 25 kV. Bis zum Erreichen der vollen Lichtausbeute vergehen nur ca. 5 Sekunden. Bis die endgültige Lichtfarbe sich eingestellt hat, können bis zu 10 Sekunden vergehen.

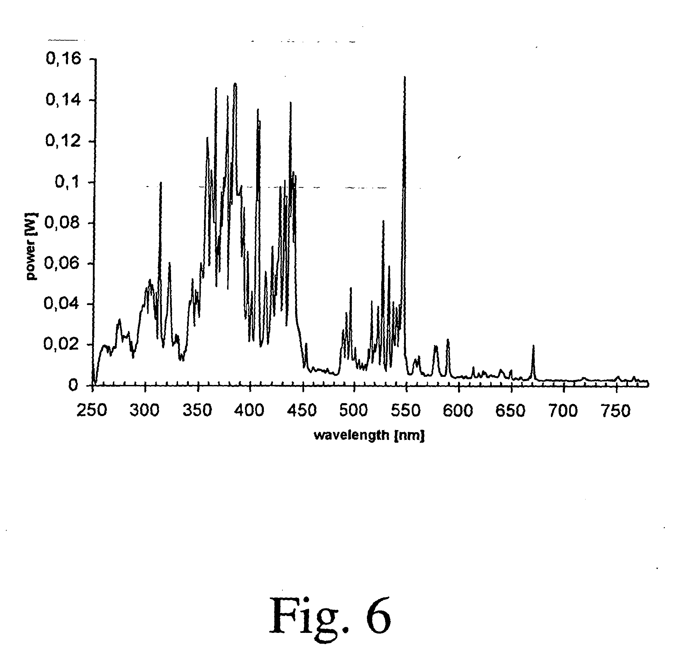

In Fig. 6 ist das Spektrum der Strahlung der Lampe 8 dargestellt. Dabei ist auf der Y-Achse die Leistung in [W] und auf der X-Achse die Wellenlänge in [nm] angegeben. Der UV/C-Bereich liegt dabei zwischen 250 bis 280 nm, der UV/B-Bereich zwischen 280 und 315 nm, der UV/A-Bereich zwischen 315 und 380 nm und der Blaulicht-Bereich zwischen 380 und etwa 500 nm. Aus der Darstellung ergibt sich, dass das Spektrum dominante, d. h. sehr hohe Spektrallinien bzw. Spektrallinienspitzen im Bereich zwischen 300 und 450 nm aufweist. Des Weiteren befinden sich Spektrallinienspitzen im Bereich zwischen 520 und 550 nm. Dieser Teil der Strahlung trägt jedoch nicht zur Bräunung bei. Darüber hinaus ist der in diesem Wellenlängenbereich emittierte Strahlungsanteil nicht größer als 40 %, insbesondere kleiner als 30 % der insgesamt emittierten Strahlung. Der überwiegende Teil der emittierten Strahlung mit mehr als 50 % liegt zwischen 280 nm und 450 nm, also im UV/A-, UV/B- und im Blaulichtbereich. In dem vorgenannten Wellenlängenbereich wird zwischen 50 % bis 80 %, insbesondere zwischen 60 % und 70 % der Strahlung emittiert.

Besondere Spektrallinienspitzen liegen zwischen 320 und 325 nm, 356 bis 359 nm, zwischen 360 und 363 nm, zwischen 364 und 367 nm, zwischen 372 und 378 nm, bei 380 nm, zwischen 381 und 391 nm, zwischen 404 und 409 nm, zwischen 430 und 432 nm, zwischen 435 und 437 nm, zwischen 438 und 442 nm.

### Bezugszeichenliste

- 1: Bräunungseinrichtung
- 2: Unterbau
- 3: Liegefläche
- 4: Oberteil
- 5: Leuchtstoff-Bräunungsleuchten
- 6: Gesichtsbräuner
- 7: Schutzscheibe
- 8: Bräunungslampe
- 9: Reflektor
- 10: Brennkammer
- 11: Brennraum
- 12: Füllgas
- 13: Elektrode
- 14: Elektrode
- 15: elektronisches Vorschaltgerät
- 16: Hüllkolben
- 17: Kühleinrichtung
- 18: Anschlußsockel

## Patentansprüche

1. Bräunungsvorrichtung (1) zur Bräunung der menschlichen Haut, mit mindestens einem Reflektor (9) und wenigstens einer als Gasentladungslampe ausgebildeten Bräunungslampe (8), wobei die Bräunungslampe (8) eine Brennkammer (10) mit einer mehr als 20%igen Durchlässigkeit für UV-Strahlung aufweist, wobei in der Brennkammer Elektroden (13, 14) und ein Quecksilber und Metallsalze aufweisendes Füllgas (12) vorgesehen sind und wobei während des Betriebs der Bräunungslampe (8) Strahlung im UV/A- und UV/B-Bereich zur Hautbräunung emittiert wird,
**dadurch gekennzeichnet,**
**dass** die Bräunungslampe (8) als Xenon-Gasentladungslampe ausgebildet ist und im Betrieb einen Betriebsdruck von mehr als 20 bar aufweist, dass das Spektrum der Strahlung dominante Spektrallinien im UV/A-Bereich, im UV/B-Bereich und im Blaulichtbereich aufweist und dass die höchsten Spektrallinienspitzen des Spektrums mit Ausnahme von Spektrallinienspitzen im Wellenlängenbereich zwischen 520 nm und 550 nm nur im UV/A-Bereich, im UV/B-Bereich und im Blaulichtbereich vorgesehen sind.

2. Bräunungsvorrichtung nach Anspruch 1, **dadurch gekennzeichnet, dass** mehr als 30 %, vorzugsweise mehr als 40 % und insbesondere mehr als 50 % der Strahlung im UV/A-Bereich, im UV/B-Bereich und vorzugsweise im Blaulichtbereich zur Bräunung emittiert wird.

3. Bräunungsvorrichtung nach Anspruch 1 oder 2, **dadurch gekennzeichnet, dass** zwischen 50 % und 90 %, insbesondere zwischen 60 % und 80 % der Strahlung im Wellenlängenbereich zwischen 280 nm und 450 nm emittiert wird.

4. Bräunungsvorrichtung nach einem der vorhergehenden Ansprüche, **dadurch gekennzeichnet, dass** der Gasfülldruck der Bräunungslampe (8) im ausgeschalteten Zustand zwischen 6 und 10 bar liegt.

5. Bräunungsvorrichtung nach einem der vorhergehenden Ansprüche, **dadurch gekennzeichnet, dass** zur Zündung des Lichtbogens der Bräunungslampe (8) ein Hochspannungsimpuls von mehr als 15 kV, vorzugsweise zwischen 20 und 40 kV vorgesehen ist.

6. Bräunungsvorrichtung nach einem der vorhergehenden Ansprüche, **dadurch gekennzeichnet, dass** die Bräunungslampe (8) mit einer Betriebsspannung zwischen 10 und 130 Volt und/oder einer Rechteckspannung, die vorzugsweise mehr als 50 Hz aufweist, betreibbar ist.

7. Bräunungsvorrichtung nach einem der vorhergehenden Ansprüche, **dadurch gekennzeichnet, dass** ein regelbares oder nicht regelbares elektronisches Vorschaltgerät (15) vorgesehen ist.

8. Bräunungsvorrichtung nach Anspruch 7, **dadurch gekennzeichnet, dass** die Bräunungslampe (8) mittels des elektronischen Vorschaltgeräts (15) dimmbar ist.

9. Bräunungsvorrichtung nach einem der vorhergehenden Ansprüche, **dadurch gekennzeichnet, dass** die Brennkammer (10) in einem äußeren, insbesondere evakuierten Hüllkolben (16) vorgesehen ist und dass der Hüllkolben (16) eine mehr als 20%ige Durchlässigkeit für UV-Strahlung aufweist.

10. Bräunungsvorrichtung nach einem der vorhergehenden Ansprüche, **dadurch gekennzeichnet, dass** die Brennkammer (10) und/oder der Hüllkolben (16) aus UV-durchlässigen Quarzglas besteht.

11. Bräunungsvorrichtung nach einem der vorhergehenden Ansprüche, **dadurch gekennzeichnet, dass** die Brennkammer (10) und/oder der Hüllkolben (16) zur Erzielung vorgegebener UV-A- und/oder UV-B-Anteile eine Beschichtung und/oder Oberflächenbearbeitung aufweist und/oder dass die Brennkammer (10) und/oder der Hüllkolben (16) teilweise, insbesondere halbseitig als Reflektor ausgebildet ist.

12. Bräunungsvorrichtung nach einem der vorhergehenden Ansprüche, **dadurch gekennzeichnet, dass** der Innendurchmesser der Brennkammer (10) zwischen 2 und 10 mm, vorzugsweise im Bereich zwischen 5 und 6 mm liegt.

13. Bräunungsvorrichtung nach einem der vorhergehenden Ansprüche, **dadurch gekennzeichnet, dass** das Füllgas (12) der Brennkammer (10) Natriumjodid und/oder Scandiumjodid als Metallsalze aufweist.

## Claims

1. Tanning device (1) for tanning human skin, having at least one reflector (9) and at least one tanning lamp (8) designed as a gas discharge lamp, the tanning lamp (8) having a combustion chamber (10) with a more than 20% transparency to UV radiation, electrodes (13, 14) and a fill gas (12) having mercury and metal salts being provided in the combustion chamber, and radiation in the UV-A and UV-B regions being emitted during operation of the tanning lamp (8) for the purpose of skin tanning, **characterized in that** the tanning lamp (8) is designed as a xenon gas discharge lamp and has an operating pressure of more than 20 bars in operation, **in that** the spectrum of the radiation has dominant spectral lines in the UV-A region, in the UV-B region and in the blue light region, and **in that**, with the exception of spectral line peaks in the wavelength region between 520 nm and 550 nm, the highest spectral line peaks of the spectrum are provided only in the UV-A region, in the UV-B region and in the blue light region.

2. Tanning device according to Claim 1, **characterized in that** for tanning purposes more than 30%, preferably more than 40% and, in particular, more than 50% of the radiation is emitted in the UV-A region, in the UV-B region and, preferably, in the blue light region.

3. Tanning device according to Claim 1 or 2, **characterized in that** between 50% and 90%, in particular between 60% and 80%, of the radiation is emitted in the wavelength region between 280 nm and 450 nm.

4. Tanning device according to one of the preceding claims, **characterized in that** the gas fill pressure of the tanning lamp (8) lies between 6 and 10 bar in the switched off state.

5. Tanning device according to one of the preceding claims, **characterized in that** a high voltage pulse of more than 15 kV, preferably between 20 and 40 kV, is provided for striking the arc of the tanning lamp (8).

6. Tanning device according to one of the preceding claims, **characterized in that** the tanning lamp (8) can be operated with an operating voltage of between 10 and 130 volt and/or a square-wave voltage which preferably exhibits more than 50 Hz.

7. Tanning device according to one of the preceding claims, **characterized in that** a controllable or non-controllable electronic ballast (15) is provided.

8. Tanning device according to Claim 7, **characterized in that** the tanning lamp (8) can be dimmed by means of the electronic ballast (15).

9. Tanning device according to one of the preceding claims, **characterized in that** the combustion chamber (10) is provided in an outer, in particular evacuated glass envelope (16), and **in that** the glass envelope (16) has a more than 20% transparency to UV radiation.

10. Tanning device according to one of the preceding claims, **characterized in that** the combustion chamber (10) and/or the glass envelope (16) consists of UV-transparent silica glass.

11. Tanning device according to one of the preceding claims, **characterized in that** the combustion chamber (10) and/or the glass envelope (16) has a coating and/or surface machine for the purpose of attaining prescribed UV-A and/or UV-B ranges, and/or **in that** the combustion chamber (10) and/or the glass envelope (16) is designed in part, preferably unilaterally, as a reflector.

12. Tanning device according to one of the preceding claims, **characterized in that** the inside diameter of the combustion chamber (10) is between 2 and 10 mm, preferably in the range between 5 and 6 mm.

13. Tanning device according to one of the preceding claims, **characterized in that** the fill gas (12) of the combustion chamber (10) has sodium iodide and/or scandium iodide as metal salts.

## Revendications

1. Dispositif de bronzage (1) pour le bronzage de la peau humaine, comprenant au moins un réflecteur (9) et au moins une lampe de bronzage (8) conçue comme lampe à décharge gazeuse, la lampe de bronzage (8) présentant une chambre de combustion (10) avec une perméabilité au rayonnement UV supérieure à 20 %, des électrodes (13, 14) et un gaz de remplissage (12) présentant du mercure et des sels métalliques étant prévus dans la chambre de combustion et du rayonnement dans la plage UV/A et la plage UV/B étant émis pour le bronzage de la peau pendant le fonctionnement de la lampe de bronzage (8),
**caractérisé en ce que**
la lampe de bronzage (8) est conçue comme lampe à décharge gazeuse à xénon et présentant en service une pression de service de plus de 20 bars, **en ce que** le spectre du rayonnement présente des lignes spectrales dominantes dans la plage UV/A, dans la plage UV/B et dans la plage de lumière bleue et **en ce que** les pointes de lignes spectrales maximales du spectre, à l'exception de pointes de lignes spectrales dans la plage de longueurs d'ondes comprise entre 520 nm et 550 nm, sont prévues uniquement dans la plage UV/A, dans la plage UV/B et dans la plage de lumière bleue.

2. Dispositif de bronzage selon la revendication 1, **caractérisé en ce que** plus de 30 %, de préférence plus de 40 % et en particulier plus de 50 % du rayonnement sont émis dans la plage UV/A, dans la plage UV/B et de préférence dans la plage de lumière bleue pour le bronzage.

3. Dispositif de bronzage selon la revendication 1 ou 2, **caractérisé en ce qu'**entre 50 % et 90 %, en particulier entre 60 % et 80 du rayonnement sont émis dans la plage de longueurs d'ondes comprise entre 280 nm et 450 nm.

4. Dispositif de bronzage selon l'une quelconque des revendications précédentes, **caractérisé en ce que** la pression de remplissage de gaz de la lampe de bronzage (8) se situe entre 6 et 10 bars dans l'état déconnecté.

5. Dispositif de bronzage selon l'une quelconque des revendications précédentes, **caractérisé en ce qu'**une impulsion de haute tension de plus 15 kV, de préférence entre 20 et 40 kV est prévue pour l'allumage de l'arc de la lampe de bronzage (8).

6. Dispositif de bronzage selon l'une quelconque des revendications précédentes, **caractérisé en ce que** la lampe de bronzage (8) peut être utilisée avec une tension de service comprise entre 10 et 130 volts et/ou une tension rectangulaire, qui est de préférence supérieure à 50 Hz.

7. Dispositif de bronzage selon l'une quelconque des revendications précédentes, **caractérisé en ce qu'**un ballast (15) électronique réglable ou non réglable est prévu.

8. Dispositif de bronzage selon la revendication 7, **caractérisé en ce que** l'intensité de la lampe de bronzage (8) peut être ajustée au moyen du ballast (15) électronique.

9. Dispositif de bronzage selon l'une quelconque des revendications précédentes, **caractérisé en ce que** la chambre de combustion (10) est prévue dans un piston enveloppant (16) extérieur, en particulier mis sous vide, et **en ce que** le piston enveloppant (16) présente une perméabilité au rayonnement UV supérieure à 20 %.

10. Dispositif de bronzage selon l'une quelconque des revendications précédentes, **caractérisé en ce que** la chambre de combustion (10) et/ou le piston enveloppant (16) est(sont) à base de verre de quartz perméable aux rayons UV.

11. Dispositif de bronzage selon l'une quelconque des revendications précédentes, **caractérisé en ce que** la chambre de combustion (10) et/ou le piston enveloppant (16) pxésente(nt) un revêtement et/ou un traitement de surface pour obtenir des fractions de UV/A et/ou UV/B prédéfinies et/ou **en ce que** la chambre de combustion (10) et/ou le piston enveloppant (16) est(sont) conçu(s) en partie, en particulier sur un demi-côté comme réflecteur.

12. Dispositif de bronzage selon l'une quelconque des revendications précédentes, **caractérisé en ce que** le diamètre intérieur de la chambre de combustion (10) se situe entre 2 et 10 mm, de préférence dans la plage comprise entre 5 et 6 mm.

13. Dispositif de bronzage selon l'une quelconque des revendications précédentes, **caractérisé en ce que** le gaz de remplissage (12) de la chambre de combustion (10) présente de l'iodure de sodium et/ou de l'iodure de scandium comme sels métalliques.
